Europäisches Patentamt

European Patent Office

Office européen des brevets

(19)

(11) Publication number: **0 150 864**
**A2**

(12) # EUROPEAN PATENT APPLICATION

(21) Application number: **85101078.5**

(22) Date of filing: **01.02.85**

(51) Int. Cl.⁴: **A 61 B 5/00**, G 01 N 27/46

(30) Priority: **02.02.84 JP 17616/84**

(43) Date of publication of application: **07.08.85**
**Bulletin 85/32**

(84) Designated Contracting States: **DE FR GB**

(71) Applicant: **SUMITOMO ELECTRIC INDUSTRIES
LIMITED, No. 15, Kitahama 5-chome Higashi-ku,
Osaka-shi Osaka-fu (JP)**

(72) Inventor: **Ohkawa, Shinichi, 1-3, Shimaya, 1-chome,
Konohana-ku Osaka (JP)**

(74) Representative: **KUHNEN & WACKER
Patentanwaltsbüro, Schneggstrasse 3-5 Postfach 1729,
D-8050 Freising (DE)**

(54) Method of applying a gas permeable membrane for blood gas sensing electrode and a device thereof.

(57) An improved method of applying a gas permeable membrane to an electrode portion of cutaneous blood gas sensor is disclosed, wherein a cushioning disk of porous material with one-sided tackified sheet of substrate, preferably having a convex-shaped surface, is utilized when the membrane is applied to the surface of the electrode portion, to which a few drops of electrolyte solution have been supplied. According to the method of the present invention, a thin, uniform layer of electrolyte solution between the membrane and electrode portion is obtained and shortened initial stabilization period, quick response, and minimized drift of the measurement are resulted.

EP 0 150 864 A2

Patentanwälte/European Patent Attorneys:
Rainer A. Kuhnen*, Dipl.-Ing.
Paul-A. Wacker*, Dipl.-Ing., Dipl.-Wirtsch.-Ing.
Wolfgang Luderschmidt**, Dr., Dipl.-Chem.

Sumitomo Electric
Industries, Ltd.
Osaka, Japan

56 SU05 12 3

## METHOD OF APPLYING A GAS PERMEABLE MEMBRANE
## FOR BLOOD GAS SENSING ELECTRODE AND A
## DEVICE THEREOF

The present invention relates to a method of applying a
gas permeable membrane for a blood gas sensing electrode
and a device thereof according to the opening part of
claims 1 and 3.

Such methods and devices are known from the following
related applications forming the prior art of the present
invention:

    (1)  CH-PS 530,006 (Eberhard, P. et al)

    (2)  DE-AS 2,758,413 (Hagihara, B.)

    (3)  U.S. Pat 4,197,853 (Parker, D.)

    (4)  DE-AS 2,540,987 (Busack, H-J; et al)

    (5)  U.S. Pat 4,324,256 (Vesteragr; P.K.R)

    (6)  Japanese Pat. Publication 60,237/81 (Ohkawa, S)
        (Japanese Pat. Application No. 157,092/79)

    (7)  Japanese Utility Model Publication 31,220/84
        (Ohkawa, S)
        (Japanese Utility Model Application
        No. 149,314/80)

    (8)  U.S. Pat 4,395,054 (Leist. H.J., et al.)

**Büro Frankfurt/Frankfurt Office:

*Büro München/Munich Office:

Adenauerallee 16    Tel. 06171/300-1
D-6370 Oberursel    Telex: 526547 pawa d

Schneggstraße 3-5    Tel. 08161/6209-1
D-8050 Freising    Telex 526547 pawa d

Telegrammadresse: Pawamuc — Postscheck München 136052-802

## DESCRIPTION OF THE PRIOR ART

Continuous measurement of the partial pressure of gases in blood of patient under critical care has been widely used. Most of the sensors for the non-invasive measurement utilize electromechanical method and are equipped with electrode assembly which is sensitive for the object gas fraction, such as oxygen, carbon dioxide, or hydrogen, respectively.

In all of the above electrode assemblies, every electrode assembly has a gas permeable membrane, which is incorporated in either at the electrode's first assembling operation or at the time of scheduled replacement of consumables such as electrolyte solution or gas permeable membrane.

A conventional cutaneous PCO2 measuring electrode assembly and the method of applying the membrane comprises a measuring glass electrode and Ag/AgCl reference electrode being placed in a housing forming an electrode portion. To the electrode portion a membrane supporting ring which holds a membrane thereabove is threaded.

During the process of applying the membrane held in the membrane supporting ring onto the electrode portion, the electrode assembly is turned over and is placed in an opposite position so that electrolyte solution is supplied dropwise on the exposed surfaces of the electrodes, followed by threading the membrane supporting ring with membrane around the counter facing electrode portion.

During the above threading procedure the membrane is pushed up by the face of the electrode portion and uneven, thick layer of electrolyte solution is resulted. This

0150864

3

causes prolonged initial stabilization period, drift of the measurement, and delayed response time of the sensor.

The problem underlying the invention is therefore to provide a method and device for applying a gas permeable membrane to an electrode portion of a blood gas monitoring electrode assembly so that the blood gas monitoring electrode assembly has a short initial stabilization period, a quick response capability and a high degree of measuring stability.

The solution of this problem is achieved by the characterizing features of claims 1 and 3.

By using a cushioning disk of porous material with one-sided tackified sheet of substrate, preferably having a convex-shaped surface, when the membrane is applied to the surface of the electrode portion, on which a few drops of electrolyte solution have been supplied, a thin, uniform layer of electrolyte solution between the membrane and electrode portion is obtained and shortened initial stabilization period, quick response, and minimized drift of measurement are resulted.

BRIEF DESCRIPTION OF THE DRAWINGS

The method of the present invention and its advantageous details will become better understood from the following description of an exemplary embodiment illustrated in the drawings, in which

Fig. 1 is a sectional view of a conventional transcutaneous blood gas sensor;

Fig. 2 illustrates the status of the electrode assembly in which a membrane supporting ring is just being threaded with electrode portion according to the method of application of membrane of the present invention.

Fig. 3 is also a sectional view of the electrode assembly which has approached the final status after the process shown in Fig. 2, with membrane supporting ring and electrode portion being completely threaded together according to the method of the present invention.

Fig. 4 is a cross-sectional view of another embodiment for a pliable cushioning device. Instead of utilizing pliable porous material, an air-cushion is used in this embodiment.

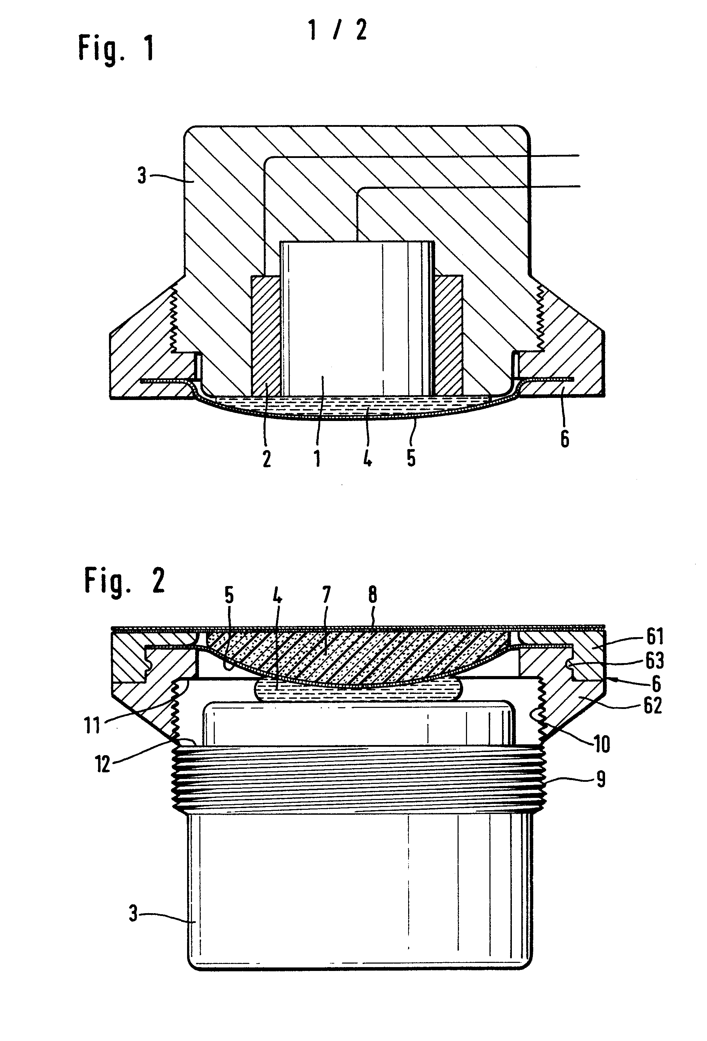

Through Fig. 1 to Fig. 4, common numerical references are used for the same elements or portions.

    1 : measuring electrode
    2 : reference electrode
    3 : electrode portion
    4 : electrolyte solution
    5 : gas permeable membrane
    6 : membrane supporting ring
    61: inner separable ring
    62: outer separable ring
    63: juncion point
    7 : cushioning disk
    8 : one-side tackified substrate
    9 : outer thread
    10: inner thread
    11: sealing plane
    12: sealing plane

13: film

14: edge portion

## DESCRIPTION OF THE PREFERRED EMBODIMENTS

Fig. 1 illustrates a conventional cutaneous PCO2 measuring electrode assembly and the method of applying a membrane is hereafter described. In the Figure a measuring glass electrode 1 and Ag/AgCl reference electrode 2, are placed in a housing forming an electrode portion 3. To the electrode portion 3 a membrane supporting ring 6 which holds a membrane 5 thereabove is threaded.

During the process of applying the membrane 5 held in the membrane supporting ring 6 onto the electrode portion 3, the electrode assembly is turned over and is placed in an opposite position so that the electrolyte solution 4 is supplied dropwise on the exposed surfaces of the electrodes 1 and 2, followed by threading the membrane supporting ring 6 with membrane around the counter facing electrode portion 3.

During the above threading procedure the membrane 5 is pushed up by the face of the electrode portion 3 and uneven, thick layer of electrolyte solution 4 is resulted. This causes prolonged initial stabilization period, drift of the measurement, and delayed response time of the sensor.

These drawbacks are remedied by the present invention.

In the following preferred embodiments of the method of applying a gas permeable membrane to cutaneous blood gas (sensor) monitoring electrode assembly are described. In Fig. 2 a membrane 5 is positioned between an inner ring 61 and an outer ring 62 of a supporting ring (6) which are combined at junction point 63. The electrode portion 3 is facing the above-described membrane 5 and electrolyte solution is supplied dropwise onto the facing surface of the electrode portion 3.

A piece of cushioning disk 7 which is made of porous polymeric matrials selected from groups of polymers such as rubber, polyurethane, polystyrene, or polyethylene, is shaped in a disk form with a convex surface at one side and a flat plane on the other side, whose surface faces a sheet of substrate such as one-side adhering paper. The substrate 8 has been adhered to the flat side of the cushioning disk 7 before application of the cushioning disk 7 to the electrode assembly.

After electrolyte solution has been supplied on the surface of the electrode portion 3 membrane supporting ring 6, which consists of two separable rings 61 and 62, is assembled with electrode portion 3 by threading at side portions 9 and 10 which are located in the inner side of the ring 62 and in the outer side of the electrode portion 3 respectively. The convex side of the cushion 7 presses the central portion of membrane onto the surface of the electrode portion 3, expelling an excess electrolyte solution out of the space surrounded by membrane 5, electrode portion 3 and membrane supporting ring 6 through threads 9 and 10.

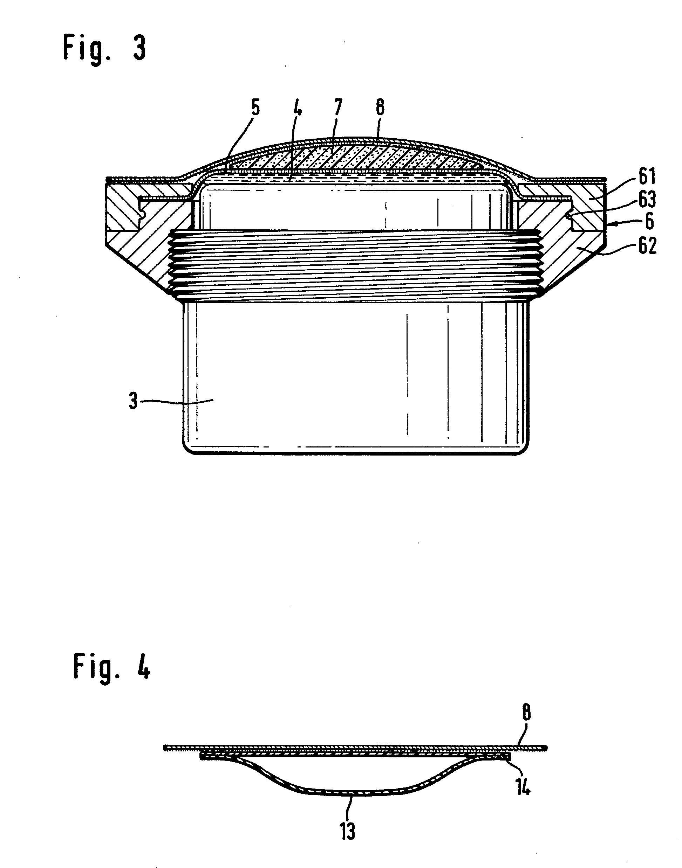

An assembled view of the sensor is illustrated in Fig. 3. The gas permeable membrane 5 is pressed onto the surface of the electrode portion 3 and drops of the electrolyte solution form a thin, uniform layer 4 without possibility of leakage of atmosperic air into the electrode portion 3. The cushioning disk 7 can easily be removed from the outer surface of the membrane by peeling the substrate 8 off the adhering surface of the supporting ring 61.

According to the method of applying a gas permeable membrane to the electrode portion, a blood gas monitoring electrode assembly with short initial stabilization period, quick response capability and with higher degree of measuring stability is achieved by providing a uniform, thin layer of electrolyte solution between the gas permeable membrane and the surface of electrode portion which faces the skin of the patient to be monitored.

In Fig. 4 another preferred embodiment of the present invention is illustrated. In this embodiment, two sheets of plastic film 13 are sealed together and the air cushion attachment can be supported by the one-side adhering substrate 8 at its edge portion 14.

It should be understood that various modifications of the above-described embodiments may be made without departing from the same scope of the present invention. For instance, blood gas monitoring sensor utilizing non-electrochemical principle such as optical absorbance measurement as disclosed in Japane Patent Publication No. 80237/81, may be improved by the method of the present invention.

Patentanwälte/European Patent Attorneys:
Rainer A. Kuhnen*, Dipl.-Ing.
Paul-A. Wacker*, Dipl.-Ing., Dipl.-Wirtsch.-Ing.
Wolfgang Luderschmidt**, Dr., Dipl.-Chem

Sumitomo Electric
Industries, Ltd.
Osaka, Japan

56 SU05 12 3

CLAIMS

1. Method of applying a gas permeable membrane to an electrode assembly for blood gas monitoring having a measuring electrode (1), a reference electrode (2), a gas permeable membrane (5), a membrane supporting portion (6) and a housing, comprising:

   means for threading the membrane supporting portion (6) with pre-attached membrane (5) to the electrode portion (3) of the assembly forcing pre-supplied drops of an electrolyte solution (4) on the surface of the electrode portion (3) facing the membrane (5) through the membrane (5) to spread over that surface of the electrode portion by a surface of cushioning disk (7) of porous elastic material, whose opposite side has been adhered to a sheet of one-side tackified substrate (8), so as to expel an excess of the electrolyte solution (4) through threads (10,11) during the process of combining the membrane supporting portion (6) and the electrode portion (6) by threading between.

2. Method of applying a gas permeable membrane to an electrode assembly for blood gas monitoring according

**Büro Frankfurt/Frankfurt Office:

Adenauerallee 16      Tel. 06171/300-1
D-6370 Oberursel      Telex: 526547 pawa d

*Büro München/Munich Office:

Schneggstraße 3-5   Tel. 08161/6209-1
D-8050 Freising      Telex 526547 pawa d

Telegrammadresse: Pawamuc — Postscheck München 136052-802

Claim 1, wherein said cushioning disk (7) has a convex surface and drops of the electrolyte solution (4) are forced to spread by means of the convex surface thereof.

3. A cushioning device for applying a gas permeable membrane to an electrode assembly for blood gas monitoring having a measuring electrode (1), reference electrode (2) and housing, characterized in that the cushioning device (7) is disk-shaped and made of porous elastic material and a surface of the disk is adhered to surface of one-side tackified substrate (8).

4. A cushioning device according to Claim 3, wherein the cushioning device (7) is made of the materials selected from such elastic porous materials as rubber, polyurethane, polysyrene and polythylene.

5. A cushioning device according to Claims 3 or 4, wherein the cushioning device (7) is a disk whose one surface is flat and the other one is convex.

0150864

1 / 2

## Fig. 1

## Fig. 2

Fig. 3

Fig. 4